# EUROPEAN PATENT SPECIFICATION

(11) **EP 3 408 264 B1**
(45) Date of publication and mention of the grant of the patent: **11.03.2020**
(21) Application number: 17701508.8
(22) Date of filing: 26.01.2017
(51) Int. Cl.: C07D 401/14, A61K 31/506, A61P 35/02

(54) **NILOTINIB DINITRATE (V) AND CRYSTALLINE FORMS THEREOF**
NILOTINIB DINITRAT (V) UND KRISTALLINE FORMEN DAVON
DINITRATE DE NILOTINIB (V) ET FORMES CRISTALLINES DE CELUI-CI

(30) Priority: 26.01.2016 SI 201600025
(43) Date of publication of application: 05.12.2018
(73) Proprietor: Farma GRS, d.o.o., 8000 Novo mesto (SI)
(72) Inventor: BENKIC, Primoz, 1000 Ljubljana (SI); MARKELJ, Mateja, 8321 Brusnice (SI); PLEVNIK, Miha, 1295 Ivancna Gorica (SI); CELIC, Tadeja Birsa, 5263 Dobravlje (SI); ZUPET, Rok, 1000 Ljublijana (SI); TIHI, Jaroslav, 8000 Novo mesto (SI)
(74) Representative: Hoefer & Partner Patentanwälte mbB
(86) International application number: PCT/EP2017/051676
(87) International publication number: WO 2017/129694

(56) References cited:
- WO-A1-2007/015871
- WO-A1-2011/163222
- US-A1- 2013 210 847
- W.L.F. Armarego, C.L.L. Chai: "purification of laboratory chemicals (5th edition)", 1 January 2003 (2003-01-01), XP055349860, pages 57-58, passage bridging p.57-58
- GOULD ET AL: "Salt selection for basic drugs", INTERNATIONAL JOURNAL OF PHARMACEUTICS, ELSEVIER, AMSTERDAM, NL, vol. 33, no. 1-3, 1 November 1986 (1986-11-01), pages 201-217, XP025813036, ISSN: 0378-5173, DOI: 10.1016/0378-5173(86)90055-4 [retrieved on 1986-11-01]

## Description

### FIELD OF THE INVENTION

Disclosed herein is a novel and stable salt of nilotinib, nilotinib dinitrate (V), its polymorphic forms as well as processes for its preparation, pharmaceutical compositions comprising it, and the preparation of such pharmaceutical compositions.

### BACKGROUND OF THE INVENTION

Nilotinib, 4-methyl-N-[3-(4-methyl-1H-imidazol-1-yl)-5-(trifluoromethyl)-phenyl]-3-[[4-(3-pyridinyl)-2-pyrimidinyl]amino]-benzamide, with the following structural formula: 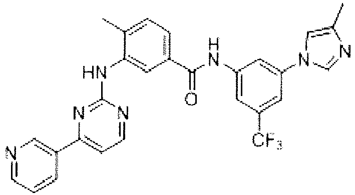 is a tyrosine kinase inhibitor used for the treatment of Philadelphia chromosome positive CML in adult patients whose disease has progressed, or who cannot tolerate therapies that include imatinib. Nilotinib is marketed in the USA and the EU under the name Tasigna® in the form of capsules comprising the hydrochloride salt of nilotinib.

WO 2007/015870 and WO 2007/015871 disclose crystalline and amorphous forms of nilotinib free base, nilotinib hydrochloride and nilotinib sulfate (VI). The crystalline forms may exist in solvate, anhydrous or hydrate forms. WO 2007/015870 describes crystalline forms of nilotinib including nilotinib HCl crystalline form A and WO 2010/054056 discloses crystalline forms of nilotinib HCl including nilotinib HCl crystalline forms T17, T18 and T19. WO 2011/033307 describes nilotinib dihydrochloride salt and a crystalline form thereof. WO2011/163222 further describes nilotinib HCl, nilotinib fumarate, nilotinib 2-chloromandelate, nilotinib succinate, nilotinib adipate, nilotinib L-tartrate, nilotinib glutarate, nilotinib p-toluenesulfonate, nilotinib camphorsulfonate, nilotinib glutamate, nilotinib palmitate, nilotinib quinate, nilotinib citrate, nilotinib maleate, nilotinib acetate, nilotinib L-malate, nilotinib L-aspartate, nilotinib formate, nilotinib HBr, nilotinib oxalate and nilotinib malonate.

In the pharmaceutical industry, there is a constant need to identify critical physicochemical parameters such as novel salts and novel polymorphic forms that affect the drug's performance, stability, etc., and which may play a key role in determining a drug's market acceptance and success. There therefore remains a need for novel salts of nilotinib with improved characteristics, as well as for novel solid state forms of such nilotinib salts.

The term "polymorphism" includes different physical forms, crystal forms, and crystalline/liquid crystalline/non-crystalline (amorphous) forms. It has been observed that many pharmaceutically active compounds exhibit polymorphism. A single molecule may give rise to a variety of polymorphs having distinct crystal structures and physical properties like melting point, thermal behaviors (e.g. measured by thermogravimetric analysis (TGA), or differential scanning calorimetry (DSC)), X-ray powder diffraction (XRPD) pattern, infrared absorption fingerprint, and solid state nuclear magnetic resonance (NMR) spectrum. One or more of these techniques may be used to distinguish different polymorphic forms of a compound. Some polymorphic forms of a given drug exhibit superior bioavailability, and consequently show much higher activity compared to other polymorphs. It is also known that the amorphous forms in a number of drugs exhibit different dissolution characteristics and in some cases, different bioavailability patterns as compared to the crystalline form. For some therapeutic indications, one bioavailability pattern may be favored over another.

Discovering new salts and new polymorphic forms and solvates of a pharmaceutical product can provide materials having desirable processing properties, such as ease of handling, ease of processing, storage stability, ease of purification or as desirable intermediate crystal forms that facilitate conversion to other polymorphic forms. New polymorphic forms and solvates of a pharmaceutically useful compound or salts thereof can also provide an opportunity to improve the performance characteristics of a pharmaceutical product. It enlarges the repertoire of materials that a formulation scientist has available for formulation optimization, for example by providing a product with different properties, e.g., better processing or handling characteristics, improved dissolution profile, improved hygroscopicity, or improved shelf-life. For at least these reasons, there still exists a need for additional nilotinib salts, and their polymorphs.

### SUMMARY OF THE INVENTION

In one aspect, provided herein is nilotinib dinitrate (V) and solid state forms of nilotinib dinitrate (V), polymorphic forms of nilotinib dinitrate (V), nilotinib dinitrate (V) having the following structural formula:

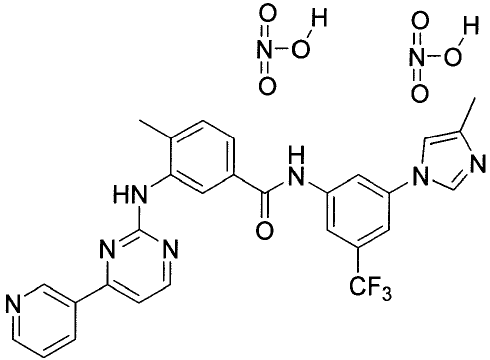

In another aspect, encompassed herein is a process for preparing a crystalline or amorphous form, hydrated and anhydro forms of nilotinib dinitrate (V) comprising contacting nilotinib free base with nitric (V) acid HNO₃ in a suitable solvent under suitable conditions to produce a reaction mass, and isolating the solid state form of nilotinib dinitrate (V). Nilotinib dinitrate (V) may also be obtained by contacting the free base of nilotinib with ammonium nitrate (V), (NH₄NO₃); or by contacting a salt of nilotinib such as e.g. nilotinib HCl, with with ammonium nitrate (V).

In another aspect, provided herein is a pharmaceutical composition comprising a solid state form of nilotinib dinitrate (V) as disclosed herein, and one or more pharmaceutically acceptable excipients.

In a still further aspect, encompassed herein is a process for preparing a pharmaceutical formulation comprising combining any one of the solid state forms of nilotinib dinitrate (V) disclosed herein with one or more pharmaceutically acceptable excipients.

In another aspect, the solid state forms of nilotinib dinitrate (V) are disclosed herein for use in the pharmaceutical compositions having particle size determined by microscopic method measuring the longest linear dimension of the particle in the range of 1 to 200 µm preferably 1 to 100 µm, most preferably 1 to 50 µm.

Nilotinib dinitrate (V) and its polymorphic forms of the present invention may be prepared directly from the isolated nilotinib base, from a reaction mixture comprising nilotinib base or by converting any nilotinib salt into its free base followed by its conversion into the dinitrate (V) salt.

### BRIEF DESCRIPTION OF THE DRAWINGS

FIG. 1 represents characteristic dynamic sorption-desorption curves for nilotinib dinitrate (V) and nilotinib HCl.
FIG. 2 is a characteristic Powder X-ray Diffraction (XRD) pattern of crystalline Form I of nilotinib dinitrate (V).
FIG.s 3a to 3s, respectively, show Powder X-ray Diffraction (XRD) pattern of nilotinib dinitrate (V) obtained according to Examples 8 to 26.

### DETAILED DESCRIPTION OF THE INVENTION

Crystalline nilotinib dinitrate (V) and various polymorphic forms thereof were produced according to the following manufacturing method(s).

Crystalline nilotinib dinitrate (V) can be produced by a process comprising the following steps:
i) dissolving nilotinib base in inert solvent or mixture of solvents at elevated temperature,
ii) adding of nitric (V) acid, optionally concentrated, preferably diluted with water, more preferably 20 wt % nitric (V) acid in water,
iii) optionally contacting the mixture under ii.) with an anti-solvent,
iv) cooling and optionally stirring the mixture under ii) or iii.) to the crystallization temperature to precipitate the product,
v) optionally, additionally cooling the suspension to lower temperatures, in particular to optimize the yield and purity of the product,
vi) isolating and drying the formed solid,
wherein seeding is optionally performed in any of the steps ii) to iv.).

The molar ratio between nitric (V) acid and nilotinib base may be between 3 to 1.5, preferably between 2.5 to 1.9, most preferably 2.1 to 1.9. It is preferred to use aqueous solutions of nitric (V) acid comprising less than 30 wt% of HNO₃, most preferably less than 20 wt% of HNO₃.

Step i) of dissolving nilotinib base in inert solvent or mixture of solvents at elevated temperature can be carried out at an elevated temperature of e.g. more than room temperature (20-24°C), in particular at a temperature or temperature range above 25°C, especially above 30°C, further especially above 45°C, still further especially above 62°C. The elevated temperature applied for dissolving nilotinib base can be e.g. the boiling temperature of the solvent or the mixture of solvents used for dissolving nilotinib base or a temperature below said boiling temperature, and can be especially e.g. a temperature or temperature range below 80°C, further especially below 70°C. The skilled person may choose an appropriate elevated temperature on the basis of the general knowledge in the art and/or the disclosure of the present application.

In step v) lower temperatures can be a temperature range below crystallization temperature, e.g. a temperature range having its higher temperature range limit at least 1 °C, especially at least 5°C, below crystallization temperature, and in particular can be a temperature or temperature range below 38°C, further in particular below 30°C, especially below 26°, further especially the temperature range of 17 - 24 °C.

The adding of nitric (V) acid in step (ii) can be an adding of an aqueous solution of nitric (V) acid. The aqueous solution of nitric (V) acid can comprise 77 wt% (weight percent) of HNO₃ or less, in particular the aqueous solution of nitric (V) acid can comprise nitric (V) acid in the range of 1 to 77 wt.-%, further in particular the aqueous solution of nitric (V) acid can comprise 68 wt% of HNO₃ or less, especially the aqueous solution of nitric (V) acid can comprise less than 60 wt% (weight percent) of HNO₃, preferably less than 30 wt% of HNO₃, more preferably less than 20 wt% of HNO₃, each based on the total weight of the aqueous solution of nitric (V) acid. The aqueous solution of nitric (V) acid can comprise e.g. at least 1 wt% of HNO₃, e.g. at least 5 wt% of HNO₃, each based on the total weight of the aqueous solution of nitric (V) acid. In particular, the aqueous solution of nitric (V) acid can comprise 77 wt% of HNO₃ or 68 wt% of HNO₃ or 27 wt% of HNO₃, each based on the total weight of the aqueous solution of nitric (V) acid.

The product precipitated in step (iv) can be or can comprise nilotinib dinitrate (V) salt, especially crystalline nilotinib dinitrate (V) salt, in particular polymorphic Form I of nilotinib dinitrate (V). The product obtained by carrying out step (v) can be or can comprise nilotinib dinitrate (V) salt, especially crystalline nilotinib dinitrate (V) salt, in particular polymorphic Form I of nilotinib dinitrate (V). The isolated and dried solid obtainable by carrying out step vi) can be or can comprise nilotinib dinitrate (V) salt, especially crystalline nilotinib dinitrate (V) salt, in particular polymorphic Form I of nilotinib dinitrate (V) (as defined herein). Crystalline nilotinib dinitrate (V) salt can be especially characterized by a Powder X-ray diffraction (XRD) pattern having peaks at: 19.7, 20.7, and 25.8 ±0.2 degrees 2-theta, in particular having peaks at: 8.9, 18.4, 19.7, 20.7, and 25.8 ±0.2 degrees 2-theta, further in particular having peaks at: 6.5, 8.9, 13.1, 15.6, 18.4, 19.7, 20.7, 23.5, and 25.8 ±0.2 degrees 2-theta.

Fig.s 3a to 3s show Powder X-ray diffraction (XRD) patterns from nilotinib dinitrate (V) salts prepared according to Examples 8 to 26 and confirm that the method of the present invention provides polymorphic Form I of nilotinib dinitrate (V).

In step i) of dissolving nilotinib base in inert solvent or mixture of solvents at elevated temperature, the inert solvent or mixture of solvents can be in particular an inert polar solvent or a mixture of inert polar solvents.

In step i) of dissolving nilotinib base in inert solvent or mixture of solvents at elevated temperature, the inert solvent or mixture of solvents can be in particular an inert organic solvent or a mixture comprising or consisting of at least two solvents selected from the group consisting of inert organic solvents and water.

In step i) of dissolving nilotinib base in inert solvent or mixture of solvents at elevated temperature, the inert solvent or mixture of solvents can be selected from the group consisting of dialkylsulfoxides, alcohols, cyclic ethers (in particular cyclic alkyl ethers), and solvent mixtures comprising or consisting of at least two solvents selected from the group consisting of dialkylsulfoxides, alcohols, cyclic ethers (in particular cyclic alkyl ethers), and water.

The total amount of organic solvents, in particular the total amount of solvents selected from the group consisting of dialkylsulfoxides, alcohols, cyclic ethers, in a mixture of solvents of step i) can be e.g. between 40 % by weight and 100 % by weight, in particular can be e.g. at least 50 % by weight, in particular at least 75 % by weight, especially at least 85 % by weight, further especially at least 95 % by weight, based on the total weight of the solvent mixture of step i). The amount of water in a solvent mixture of step i) can be e.g. between 0 % by weight and 60 % by weight, e.g. can be less than 50 % by weight, especially less than 30 %, further especially less than 25 % by weight, still further especially less than 10 % by weight, based on the total weight of the mixture of solvents of step i).

In particular, step i) can be dissolving nilotinib base in
- inert solvent selected from the group consisting of dimethylsulfoxide, and alcohols (in particular C2-C4 alkan(di)ols (e.g. propandiol (e.g. 1,2-propandiol)) and alkan(mono)ols (e.g. C1-C4 alkan(mono)ols, e.g. methanol),
   or can be dissolving nilotinib base in
- a solvent mixture selected from the group consisting of
- solvent mixture comprising or consisting of dimethylsulfoxide and at least one alcohol (preferably at least one C1-C5 alkyl alcohol, more preferably at least one C1-C4 alkyl alcohol, e.g. methanol),
- solvent mixture comprising or consisting of dimethylsulfoxide and water,
- solvent mixture comprising or consisting of dimethylsulfoxide and at least one alcohol (preferably at least one C1-C5 alkyl alcohol, more preferably at least one C1-C4 alkyl alcohol, in particular methanol), and water,
- solvent mixture comprising or consisting of cyclic ether(s) (especially cyclic alkyl ether(s), in particular tetrahydrofuran) and water,
- solvent mixture comprising or consisting of at least one alcohol (especially butanol, in particular 1-butanol) and water.

The alcohol(s) used in step i) as inert solvent or as solvent(s) in the solvent mixture can be in particular alkyl alcohol, especially alkyl alcohol which can comprise at least one (e.g. 1, 2, or 3) OH-group(s), especially (e.g. straight chain or branched or cyclic) C1-C5 alkyl alcohol, preferably C1-C4 alkyl alcohol. The alcohol(s) used in step a) as inert solvent or as solvent(s) in the solvent mixture can be in particular chosen from alkane(mono)ols, alkanediols, alkanetriols, and mixtures thereof. An alkane(mono)ol can be in particular chosen from C1-C5 alkane(mono)ols, such as methanol, ethanol, propanol (e.g. 1- or 2-propanol and mixtures thereof), butanol (e.g. n-, iso-, sec-, tert-butanol, and mixtures thereof), pentanol (e.g. 1-pentanol, and all further structural isomers of pentanol, and mixtures thereof). An alkanediol (in particular a C1-C5 alkanediol) can be a vicinal alkanediol, in particular a 1,2-alkanediol, preferably 1,2-propandiol. An alkanediol may be in particular chosen from HO-C(H)(R^{a})-C(H)(OH)-R^{b}, wherein R^{a} can be C1-C2 alkyl or H, especially one of H, methyl, and ethyl, and wherein R^{b} can be C1-C2 alkyl or H, especially one of H, methyl, and ethyl.

The dialkylsulfoxide(s) used in step i) as inert solvent or as solvent(s) in a mixture of solvents can be in particular selected from dialkylsulfoxides of formula R¹-S(O)-R², wherein R¹ and R² can be each independently selected from methyl and ethyl, especially the dialkylsulfoxide can be dimethyl sulfoxide (DMSO).

The cyclic ether(s) used in step i) as inert solvent or as solvent in a mixture of solvents can be in particular cyclic alkyl ether(s), in particular tetrahydrofuran (THF).

In particular, in step i) the inert solvent or mixture of solvents can be selected from the group consisting of dimethylsulfoxide, methanol, butanol (preferably 1-butanol), propandiol (preferably 1,2-propandiol), tetrahydrofuran, and mixtures thereof, and solvent mixtures comprising or consisting of at least two solvents selected from the group consisting of dimethylsulfoxide, methanol, butanol (preferably 1-butanol), propandiol (preferably 1,2-propandiol), tetrahydrofuran, and water.

Further in particular, in step i) the inert solvent or mixture of solvents can be a mixture of THF and water. Applying a mixture of THF and water in step i) provides advantageous procedural results. Optionally, when applying a mixture of THF and water in step i), an optional step of contacting the mixture under ii.) with (additional) water can be carried out after step ii.).

In processes of the present invention, the molar ratio nilotinib base (in particular of nilotinib base dissolved in step (i)) : nitric (V) acid (in particular of nitric (V) acid added in step ii)) can be e.g. about 1:1.9 to about 1:2.3, especially about 1:1.95 to about 1:2.05, in particular about 1:2.

The anti-solvent (for use in step iii) can be an anti-solvent selected from alcohols, ketones, ethers, esters, hydrocarbons, water, and mixtures thereof.

The alcohol(s) (for use in step iii)) can be selected from the group consisting of alkyl alcohols and mixtures thereof,
especially can be selected from the group consisting of C1-C4 alkyl alcohols (which can be e.g. straight chain or branched C1-C4 alkyl alcohols) and mixtures thereof,
further especially can be selected from the group consisting of C1-C4 alkan(mono)ols (which can be e.g. straight chain or branched), and mixtures thereof,
in particular can be selected from the group consisting of methanol, ethanol, propanol (in particular 2-propanol), and mixtures thereof.
The ketone(s) (for use in step iii)) can be selected from the group consisting of dialkyl ketones, in particular can be selected from the group consisting of dialkyl ketones of formula R^{c}-C(O)-R^{d}, wherein R^{c} and R^{d} can be each independently selected from (e.g. straight chain or branched) C1-C6 alkyl, such as methyl, ethyl, propyl (such as n-propyl, iso-propyl), butyl (such as n-butyl, iso-butyl, tert-butyl), pentyl, hexyl, especially can be selected from the group consisting of ethylmethylketone, methylisobutylketone, and acetone.
The ester(s) (for use in step iii)) can be selected from the group consisting of alkyl esters, especially can be selected from the group consisting of esters of formula R^{es}-C(O)-O-R^{es'}, wherein R^{es} can be a C1-C6 alkyl (which may be e.g. straight chain or branched), especially one selected from the group consisting of methyl, ethyl, propyl, butyl, pentyl, hexyl, and wherein R^{es'} can be a C1-C6 alkyl (which may be e.g. straight chain or branched), in particular can be selected from the group consisting of methyl, ethyl, propyl, butyl, pentyl, hexyl; further especially ester(s) can be selected from the group of alkylacetates, e.g. can be selected from ethylacetate and isopropylacetate.
The ether(s) (for use in step iii)) can be selected from the group consisting of dialkyl ethers, cyclic ethers, and mixtures thereof;
in particular ether(s) can be selected from dialkyl ethers of formula R^{f}-O-R^{g}, wherein R^{f} and R^{g} can be each independently selected from (e.g. straight chain or branched) C1-C6 alkyl (such as methyl, ethyl, propyl (such as n-propyl, iso-propyl), butyl (such as n-butyl, iso-butyl, tert-butyl), pentyl, hexyl), cyclic alkyl ethers, and mixtures thereof;
especially the ether(s) can be selected from methyl tert-butyl ether, diisopropylether, diethylether, tetrahydrofuran (THF) and mixtures thereof.
The hydrocarbon(s) (for use in step iii)) can be selected from the group of alkanes, cycloalkanes, aromatic hydrocarbons and mixtures thereof. Alkanes (e.g. hexane, heptane) can comprise straight chain alkanes, branched alkanes, and mixtures thereof. Alkanes can be compounds of formula CₙH₂ₙ₊₂, wherein n can be an integer and can be e.g. 5, 6, 7, 8 or 9.
Alkanes can be especially selected from the group of straight chain alkanes, branched alkanes, and mixtures thereof,
further especially can be selected from the group of C5 to C9 alkanes and mixtures thereof, in particular C5 to C9 straight chain alkanes, C5 to C9 branched alkanes, and mixtures thereof. In particular, hexane (including straight chain hexane, branched hexanes, and mixtures thereof) or heptane (including straight chain heptane, branched heptanes, and mixtures thereof) can be used as alkane.
Cycloalkanes can be especially selected from the group of C5 to C10 cycloalkanes (e.g. cyclohexane), and mixtures thereof.
Aromatic hydrocarbons can be especially selected from the group of benzene, and mono-alkyl-substituted benzenes, di-alkyl-substituted benzenes, and mixtures thereof. Mono-alkyl-substituted benzene(s) can be in particular of formula C₆H₅-R^{h} , wherein R^{h} can be selected from (e.g. straight chain or branched) C1-C4 alkyl, such as methyl, ethyl, propyl (such as n-propyl, iso-propyl), butyl (such as n-butyl, iso-butyl, tert-butyl), especially can be toluene. Di-alkyl-substituted benzene(s) can be in particular of formula Rⁱ-C₆H₅-R^{k}, wherein Rⁱ and R^{k} can be each independently selected from (e.g. straight chain or branched) C1-C4 alkyl, such as methyl, ethyl, propyl, butyl; the substituents Rⁱ and R^{k} can be in position ortho or meta or para to each other. Especially, the hydrocarbon(s) (for use in step iii)) can be selected from hexane (which can be straight chain hexane, branched hexanes, and mixtures thereof), heptane (which can be straight chain heptane, branched heptanes, and mixtures thereof), cyclohexane, toluene and mixtures thereof.

The process for the preparation of nilotinib dinitrate (V) can be in particular a process wherein in step i) the inert solvent or mixture of solvents is selected from the group consisting of dialkylsulfoxides, alcohols, cyclic ethers (especially cyclic alkyl ethers), and solvent mixtures comprising or consisting of at least two solvents selected from the group consisting of dialkylsulfoxides, alcohols, cyclic ethers (especially cyclic alkyl ethers), and water, and
wherein the anti-solvent used in step iii) is an anti-solvent selected from the group of alcohols, ketones, ethers, esters, hydrocarbons, water, and mixtures thereof.

Particularly advantageous procedural results can be obtained for processes, wherein optional step iii) can be optionally not carried out and wherein in step (i) the inert solvent or mixture of solvents is selected from the group of: dimethylsulfoxide, methanol, propandiole (in particular 1,2-propanediol), solvent mixture comprising or consisting of butanol (especially 1-butanol) and water, solvent mixture comprising or consisting of dimethylsulfoxide (DMSO) and methanol and optionally water, solvent mixture comprising or consisting of DMSO and water, and solvent mixture comprising or consisting of tetrahydrofurane and water.

Furthermore, particularly advantageous procedural results can be obtained for processes, wherein optional step iii) is carried out (i.e. is a non-optional step), and wherein in step (i) the inert solvent or mixture of solvents is DMSO or mixture comprising or consisting of DMSO and water, and in step (iii) the anti-solvent is selected from the group consisting of methanol, ethanol, propanol (especially 2-propanol), acetone, ethylmethylketone, methylisobutylketone, diethyl ether (Et₂O), diisopropyl ether (iPr₂O), methyl *tert*-butyl ether, tetrahydrofuran (THF), ethyl acetate (EtOAc), isopropyl acetate (iPrOAc), hexane (which can be straight chain hexane, branched hexanes, and mixtures thereof), cyclohexane, heptane (which can be straight chain heptane, branched heptanes, and mixtures thereof), toluene (PhMe), water, and mixtures thereof.

In an embodiment, the anti-solvent (used in step iii) can be a solvent or mixture of solvents which when added in step (iii) results in a precipitation of the product in step (iv). In particular, the anti-solvent of step (iii) can be a solvent or a mixture of solvents different from the inert solvent or the mixture of solvents used in step (i) for dissolving nilotinib base.

According to one embodiment, the term "inert solvent" can have the identical meaning as "solvent".

In one aspect, there is provided the use of nitric (V) acid, in particular the use of an aqueous solution of nitric (V) acid, for the preparation of nilotinib dinitrate (V), in particular crystalline nilotinib dinitrate (V), further in particular polymorphic Form I of nilotinib dinitrate (V) (as defined herein), from nilotinib base.

Crystallization temperature, which according to the present invention means the temperature at which the formation of crystals of the product takes place, can be between 0 °C and up to the reflux temperature of the reaction mixture, preferably between 10°C to 80°C, most preferably between 20 °C and 60°C.

In case of remaining insoluble material in stage i) the same or different solvent(s) are added, or the mixture is heated to a temperature between room temperature and the boiling point of the solution until all solid phase is dissolved and remains dissolved upon cooling. When insoluble material is still present in spite of dilution and/or heating, the mixture can be filtered in order to remove it before crystallization or precipitation of the salt.

The resulting crystals or precipitates are usually collected by filtration or centrifugation and washed with a suitable solvent. Normally, crystallizing of the salt at lower temperature and/or cooling the mixture more rapidly results in smaller particles compared to when crystallization is initiated at higher temperatures and/or is cooled gradually. In case higher purity is desirable, the person skilled in the art may employ any of the conventional manners of re-crystallization.

Suitable inert solvents to be used in step i) and ii) are for example DMSO and/or methanol. When DMSO is used as the solvent, one of the suitable anti-solvents is e.g. methanol.

Nilotinib dinitrate (V) may also be obtained by contacting the free base of nilotinib with ammonium nitrate (V), (NH₄NO₃); particularly with a solution of ammonium nitrate (V); or by contacting a salt of nilotinib, such as e.g. nilotinib HCl, with with ammonium nitrate (V).

The solubility of nilotinib or its salt should be relatively higher in the solvent used under i) than in the optionally used anti-solvent used under iii.). In that way filtration step after formation of the salt is feasible as required for industrial production. Anti-solvents in step iii.) are optionally used to initiate crystallization, to improve yield and most importantly to design particle properties as required for good processibility, purity and formulation technology. For same purpose seeding can be performed to control crystallization in any steps from ii) to iv). Additionally and optionally solubility of nilotinib dinitrate (V) can be decreased by addition of anti-solvent that makes possible to use seeding at elevated temperatures to improve bulk properties of the isolated material.

As used herein, "reflux temperature" means the temperature at which the solvent or solvent system refluxes or boils at atmospheric pressure.

Nilotinib free base (solid form, oily form or solution in the specified solvents, in a reaction mixture or in a diluted reaction mixture) as used in the preparation of nilotinib dinitrate (V) of the present invention may be produced according to any of the reported manufacturing method, or any method employed by the person skilled in the art.

In case nilotinib base of increased purity is desired, the preparation of nilotinib dinitrate (V) may also be used as a purification step, and the nilotinib dinitrate (V) then converted to nilotinib base or another nilotinib salt. The purity of the obtained nilotinib dinitrate (V) is higher than 99%, preferably higher than 99.5 %.

The product purity was assessed by ultra performance liquid chromatography (UPLC) with a column of the type Titan C-18 100 x 2.1 mm i.d., 1.9 µm particles; column temperature: 25°C; detector: UV 254 nm; flow rate: 0.25 ml/min; injection volume: 0.5 µl; mobile phase: A: 0.15% trifluoroacetic acid solution; B: acetonitrile; Gradient: 0'=10%B, 13'-17'=98%B, 18'-20'=10%B. This UPLC method was generally applicable for the analysis of nilotinib, chromatographic purity and assay.

### Sample preparation:

Chromatographic purity: The sample solution was prepared in concentration about 0.5 mg/ml.
Dilution solvent was DMSO.
Assay: The sample solution was prepared in concentration about 0.1 mg/ml. Dilution solvent was DMSO.

### Calculation:

Chromatographic purity: Area per cent method.
Assay: External standard method

The isolated material containing nilotinib dinitrate (V) is dried in a state of the art dryer such as a fluid bed dryer, a tray dryer with or without vacuum, or a rotating dryer (application of air flow). In some cases higher humidity of inlet air is desired in order to prevent too fast drying of the surface of the material in the dryer resulting in forming a strongly impermeable layer on the surface of the particles causing slow and incomplete removal of solvent from the interior of the particles.

The obtained nilotinib dinitrate (V) particle size can be controlled by the disclosed process in the range of 1 to 200 µm, preferably 1 to 100 µm, most preferably 1 to 50 µm. Primary particles in the formed agglomerates are approximately less than 10 µm, preferably less than 5 µm in length as determined by microscopic method. Additionally milling can be performed to further reduce or de-agglomerate particles of nilotinibe dinitrate (V). State of the art milling systems may be applied like micronisation, pin mill, ball mill sifter, etc. The specific surface area of such particles can be in the range of 2 to 20, preferably 4 to 15 m²/g, calculated from the nitrogen adsorption isotherm by applying the BET theory. The particle size of nilotinib dinitrate (V) can be reduced and the particle size distribution can be narrowed, if advantageous for the manufacturing of solid dosage forms, e.g. by conventional milling or grinding methods and equipment such as jet mills and/or hammer mills. For formulation processes good flowability of material is important especially in case of direct compression formulation.

Along with other advantageous physical properties of nilotinibe dinitrate (V) most preferable for preparing a pharmaceutical composition comprising it are its low hygroscopity, low bulk volume, excellent flowability and improved solubility. It has surprisingly been found that the hygroscopicity of nilotinib dinitrate (V) is very low, particularly compared to some other nilotinib salts, such as the hydrochlorid salt of nilotinib. It is of high importance that a salt of an active pharmaceutical ingredient is stable in a wide humidity range during the formulation process (weighting, granulation, etc) because this makes the handling of the active pharmaceutical ingredient as well as the handling of mixtures comprising it much easier.

The low hygroscopicity effect of nilotinib dinitrate (V) and nilotinib HCl is shown in Table 1 and Table 2.

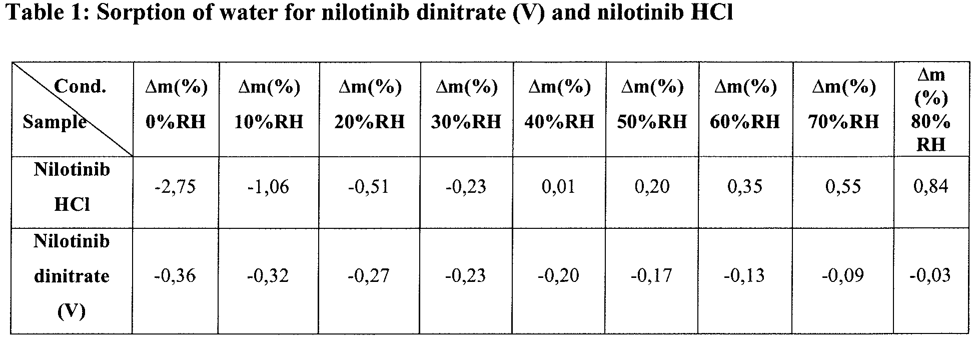

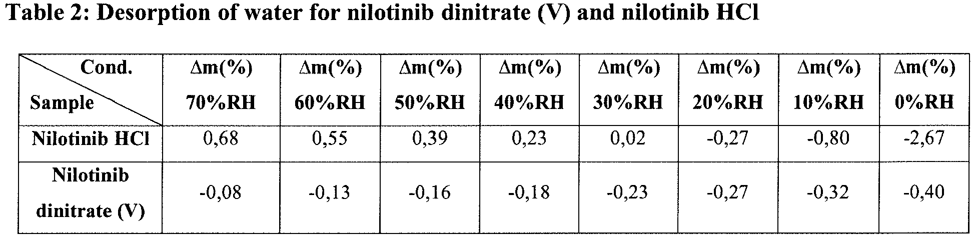

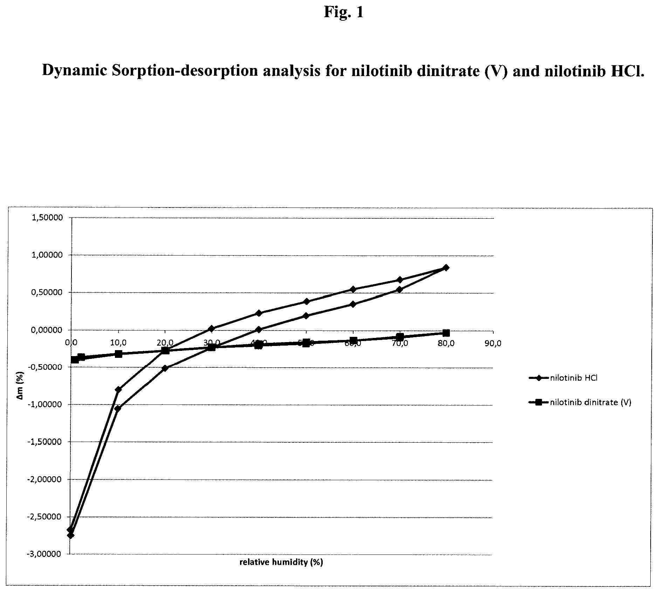

The moisture sorption data of nilotinib dinitrate (V) and nilotinib HCl as represented in Table 1 and Table 2 are also shown in Figure 1.

Another aspect of the present invention is the new polymorphic Form I of nilotinib dinitrate (V), characterized by a Powder X-ray diffraction pattern having peaks at the following 2-theta degrees ±0.2 (Table 3):

**Table 3:**

| No. | Pos. [°2Th.] | Rel. Int. [%] |
|---|---|---|
| 1 | 6.5 | 36 |
| 2 | 8.9 | 66 |
| 3 | 13.1 | 26 |
| 4 | 15.6 | 28 |
| 5 | 18.4 | 51 |
| 6 | 19.7 | 96 |
| 7 | 20.7 | 98 |
| 8 | 23.5 | 37 |
| 9 | 25.8 | 100 |

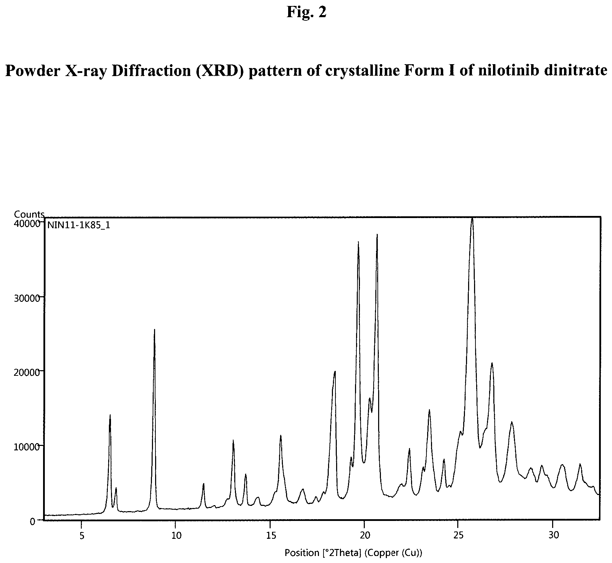

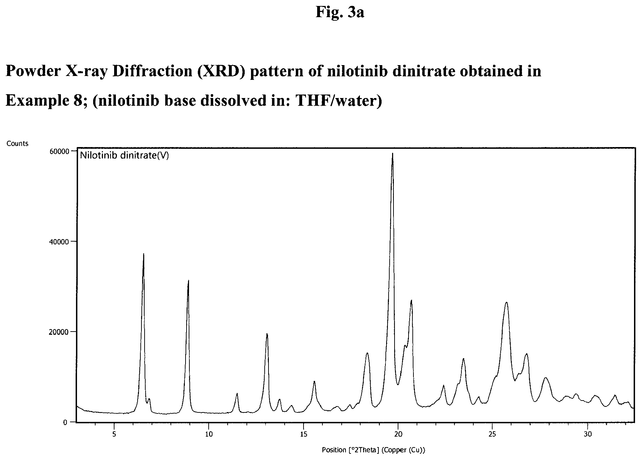

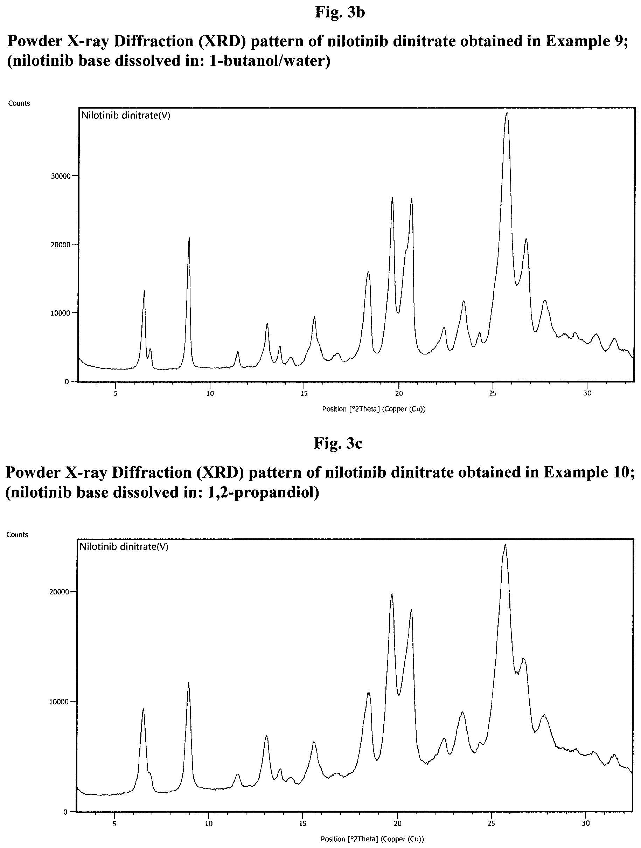

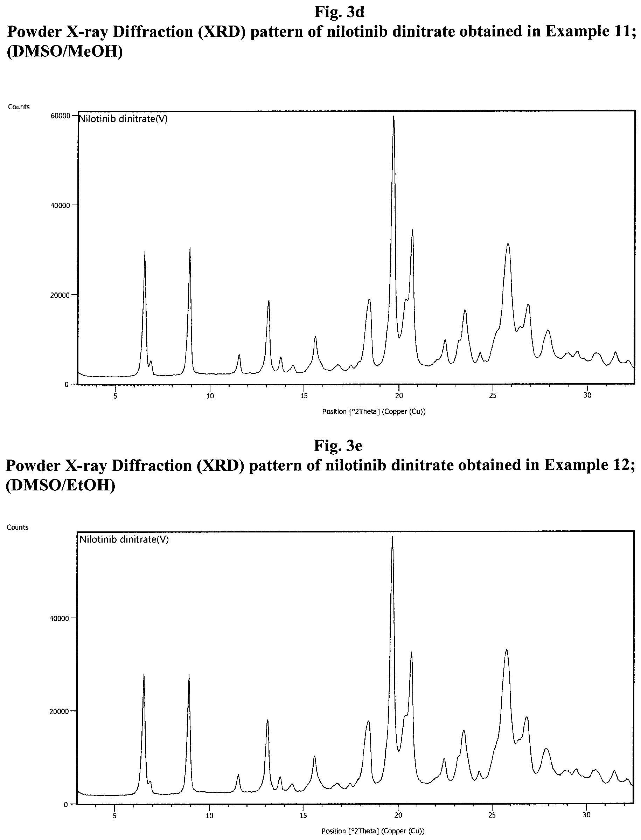

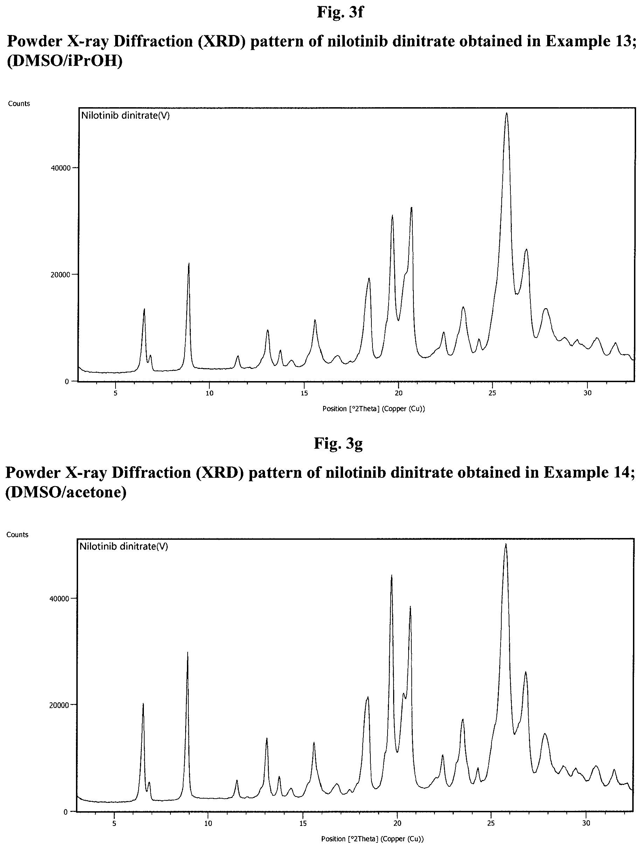

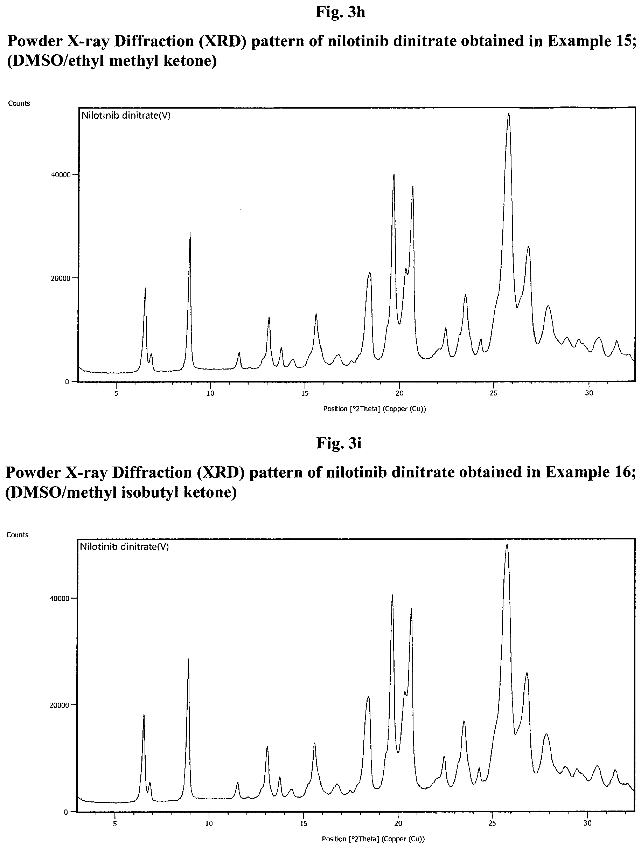

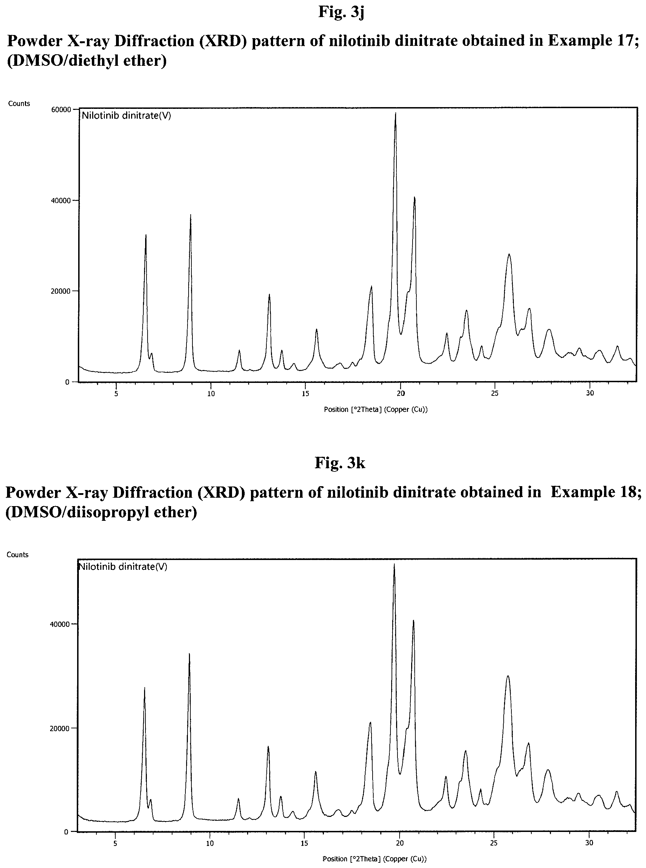

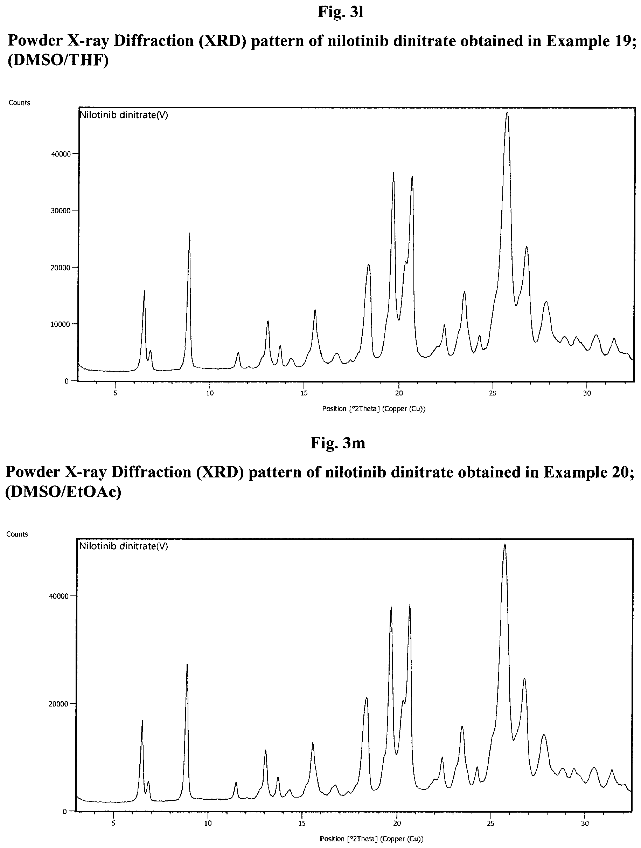

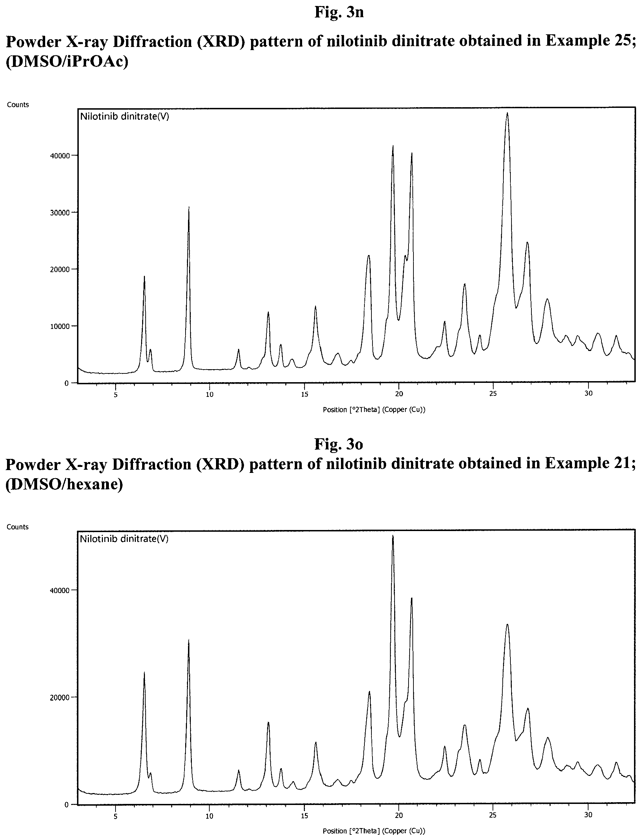

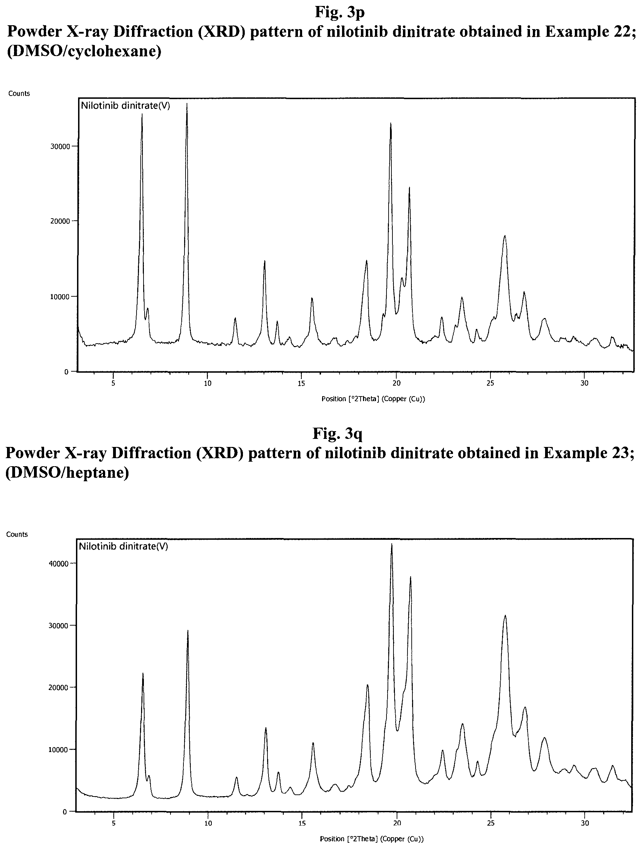

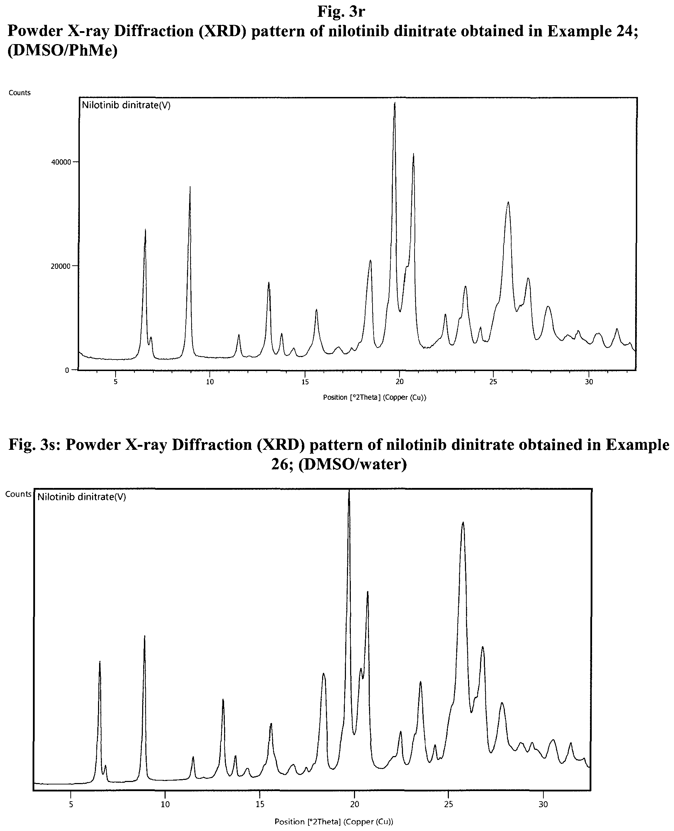

Polymorphic Form I of nilotinib dinitrate (V) is further characterized by a Powder X-ray Diffraction (XRD) pattern substantially in accordance with Fig. 2. Form I can be prepared according to the process for preparation of nilotinib dinitrate (V) according to the present invention.

The X-ray powder diffraction patterns were obtained by PANalytical X'Pert PRO diffractometer with Cu Kα radiation (λ = 1.5418 Å) at 45 kV and 40 mA and X' Celerator detector..

Nilotinib dinitrate (V) of the invention can be used as the drug substance in production of pharmaceutical preparations by combining it with conventional pharmaceutical carriers or diluents employed in this field. The pharmaceutical preparations may be produced by a method usually employed in this field.

The pharmaceutical preparations containing nilotinib dinitrate (V) of the present invention include orally administrable preparations such as tablets, pills, capsules, granules, powders, liquids and solutions, and the like; or parenteral preparations such as intravenous, or intramuscular injections, suppositories, percutaneous liquid preparations, ointments, transdermal stickers, transmucosal liquid preparations, transmucosal stickers, inhalations, and the like. Particularly, tablets, pills, capsules, granules and powders, are advantageous as stable solid preparations.

In the solid compositions for use in oral administration, one or more of the active ingredients may be mixed with at least one inert diluent, for example, lactose, mannitol, glucose, hydroxypropylcellulose, microcrystalline cellulose, powdered cellulose starch, polyvinylpyrrolidone, magnesium metasilicate aluminate, cyclodextrins and the like. The compositions may contain pharmaceutically acceptable additives other than diluents in a conventional manner, for example, lubricants such as magnesium stearate, sodium stearyl fumarate, hydrogenated castor oil and the like, disintegrating agents such as starch, fibrous calcium glycolate, sodium starch glycolate croscarmellose sodium, crospovidone and the like, stabilizers, or solubilizing agents. The tablets or pills if required may be coated with sugar-coating or a polymeric coating film, such as sucrose, gelatin, hydroxypropylcellulose, hydroxypropylmethylcellulose, and the like. The coating can additionally contain at least one pharmaceutically acceptable additive, selected from colouring agents, opacifiers, antiadherents, and the like.

The methods of preparing the solid oral dosage forms can be e.g. further be described as:

### DIRECT COMPRESSION:

Excipients are mixed in an appropriate mixer such as a biconical mixer. Nilotinib dinitrate (V) is added and mixed with excipients. To the obtained mixture the lubricant is added, homogenous mixture is finally mixed and compressed on tableting machine. Tablets can be coated with a film coating.

### WET GRANULATION:

Excipients with the exception of the lubricant are mixed. Nilotinib dinitrate (V) is added and mixed with excipients and granulated with granulation liquid. To the obtained granulate magnesium stearate is added, homogenous mixture is finally mixed and compressed on tableting machine. Water, organic solvents miscible with water such as ethanol, isopropanol, methanol, acetone or mixtures thereof can be used as a granulation liquid, in which optional binder or other excipients can be dispersed. Wet granulation can be performed by the state of the art processes and equipment such as low or high shear mixer or fluid bed granulators (top spray, bottom spray or tangential spray). The granulate may be screened and/or milled to obtain a blend with suitable technological properties/sizes. Tablets can be coated with a film coating

### DRY GRANULATION

Excipients except the lubricant are mixed. Nilotinib dinitrate (V) is added and mixed with excipients. To the obtained mixture a part of the lubricant is added. Homogenous mixture is finally mixed and dry granulated via slugging or roller compaction. Obtained slugs or granules can be further milled to achieve the appropriate size of granules. Granules of desirable size are mixed with the rest part of the lubricant and compressed into tablets, which can be additionally film coated.

According to an aspect, there is provided nilotinib dinitrate (V) salt for use as a medicament. In particular, there is provided crystalline nilotinib dinitrate (V) salt for use as a medicament. Furthermore, there is provided a hydrated or anhydrous crystalline form of nilotinib dinitrate (V) for use as a medicament. Especially, there is provided polymorphic form I of nilotinib dinitrate (V) salt, as defined herein, for use as a medicament. Crystalline nilotinib dinitrate (V) salt for use as a medicament can be especially characterized by a Powder X-ray diffraction (XRD) pattern having peaks at: 19.7, 20.7, and 25.8 ±0.2 degrees 2-theta, in particular having peaks at: 8.9, 18.4, 19.7, 20.7, and 25.8 ±0.2 degrees 2-theta, further in particular having peaks at: 6.5, 8.9, 13.1, 15.6, 18.4, 19.7, 20.7, 23.5, and 25.8 ±0.2 degrees 2-theta.

According to a further aspect, there is provided a pharmaceutical composition comprising a nilotinib dinitrate (V) salt and one or more pharmaceutically acceptable excipients. Nilotinib dinitrate (V) salt can be crystalline nilotinib dinitrate (V) salt, especially can be a hydrated or anhydrous crystalline form of nilotinib dinitrate (V) or a mixture thereof. In particular, nilotinib dinitrate (V) salt can be polymorphic form I of nilotinib dinitrate (V) salt, as defined herein. In particular, the pharmaceutical composition can be a solid pharmaceutical composition for oral administration and can comprise nilotinib dinitrate (V) salt and one or more pharmaceutically acceptable excipients, preferably the solid pharmaceutical composition for oral administration comprises at least one diluent.

In particular, the following aspects of the present invention are provided: 1. Nilotinib dinitrate (V) salt. 2. Nilotinib dinitrate (V) salt according to item 1, characterized in that it is crystalline. 3. A hydrated or anhydrous crystalline form of nilotinib dinitrate (V) according to any of items 1 to 3. 4. Crystalline nilotinib dinitrate (V) according to any of items 2 to 4, characterized by a Powder X-ray diffraction (XRD) pattern having peaks at the following 2-theta degrees: 19.7, 20.7, and 25.8 ±0.2. 5. Crystalline nilotinib dinitrate (V) according to any of items 2 to 5, further characterized by a Powder X-ray diffraction (XRD) pattern having peaks at the following 2-theta degrees: 8.9, 18.4, 19.7, 20.7, and 25.8 ±0.2. 6. Crystalline nilotinib dinitrate (V) according to any of items 2 to 6, further characterized by a Powder X-ray diffraction (XRD) pattern having peaks at the following 2-theta degrees: 6.5, 8.9, 13.1, 15.6, 18.4, 19.7, 20.7, 23.5, and 25.8 ±0.2. 7. A process for the preparation of nilotinib dinitrate (V) comprising the following steps:
(i) dissolving nilotinib base in inert solvent or mixture of solvents at elevated temperature,
(ii) adding of nitric (V) acid, optionally concentrated, preferably diluted with water, more preferably 20 wt % nitric (V) acid in water,
(iii) optionally contacting the mixture under ii.) with an anti-solvent,
(iv) cooling and optionally stirring the mixture under ii) or iii.) to the crystallization temperature to precipitate the product,
(v) additionally cooling the suspension to lower temperatures to optimize the yield and purity of the product,
(vi) isolating and drying the formed solid,
wherein seeding is optionally performed in any of the steps ii) to iv). 8. A process for the preparation of nilotinib dinitrate (V) from nilotinib base wherein DMSO and/or MeOH are used as solvent or antisolvent.

In one aspect, there is provided a process for the preparation of nilotinib dinitrate (V) comprising the following steps:
(i) dissolving nilotinib base in a mixture comprising or consisting of THF and water at elevated temperature, e.g. at a temperature in the temperature range 61°C-70°C, in particular at a temperature in the temperature range 66°C-70°C,
(ii) adding of nitric (V) acid, optionally concentrated, preferably diluted with water;
   preferably said adding of nitric (V) acid can be an adding of an aqueous solution of nitric (V) acid comprising 77 wt% of nitric (V) acid or less, in particular can be an adding of an aqueous solution of nitric (V) acid comprising nitric (V) acid in the range of 1 to 77 wt.-%, more preferably can be an adding of an aqueous solution of nitric (V) acid comprising 27 wt% of nitric (V) acid or less, still more preferably 20 wt% of nitric (V) acid or less, especially 15 wt% of nitric (V) acid or less, each based on the total weight of the aqueous solution of nitric (V) acid,
   preferably the molar ratio nilotinib base (in particular of nilotinib base dissolved in step (i)) : nitric (V) acid (in particular of nitric (V) acid added in step ii)) can be e.g. about 1:1.9 to about 1:2.3, especially about 1:1.95 to about 1:2.05, further especially about 1:2,
(iii) optionally contacting the mixture under ii.) with water,
(iv) cooling and optionally stirring the mixture under ii) or iii.) to the crystallization temperature to precipitate the product, e.g. cooling and optionally stirring the mixture under ii) or iii.) to a temperature of e.g. at least 5°C below the elevated temperature of step (i), in particular cooling and optionally stirring the mixture under ii) or iii.) to a temperature of 60°C or less,
(v) optionally, additionally cooling the suspension to lower temperatures, in particular to optimize the yield and purity of the product, (e.g. cooling the suspension to a temperature of 50°C or less, especially to a temperature in the range of 18 to 25°C),
(vi) isolating and drying the formed solid (e.g. drying at a temperature between 25 and 55°C, optionally at a pressure of less than 101325 Pascal),
wherein seeding is optionally performed in any of the steps ii) to iv).

Step (i) of dissolving nilotinib base in a mixture comprising or consisting of THF and water at elevated temperature can comprise the following steps: suspending nilotinib base in THF at elevated temperature, adding water to the suspension obtained by suspending nilotinib base in THF at elevated temperature, dissolving nilotinib base present in not dissolved form in said suspension.

In processes comprising a step (i), which is step (i) of dissolving nilotinib base in a mixture comprising or consisting of THF and water at elevated temperature, the mixture can contain THF and water in a weight ratio THF:water in the range of from 5:1 to 1:1, especially 4:1 to 3:1.

The invention is explained specifically by the following examples which are not intended as a limitation thereof and are not intended to restrict the scope of the invention.

In the examples "room temperature" is defined between 20 - 24 °C. Also, "laboratory atmosphere" is defined as air with relative humidity between 20 - 60 % and with room temperature (defined above).

"Water content in obtained product" defines the content of water in the obtained products (w/w %) as determined using Karl Fischer titration.

### EXAMPLES

### Example 1

Nilotinib base (10 g) was suspended in dimethyl sulphoxide (DMSO) - methanol mixture in a ratio of 1/1 (225 mL). The suspension was heated to 65 °C until a clear solution was obtained, and filtered. To the clear solution 2 equivalents of 20% nitric (V) acid in water (10 g) were added. The obtained solution was cooled to 60 °C, at which the material started to crystallize. The suspension was further cooled to 20 °C, and filtered under vacuum. The wet material was dried under vacuum at 55 °C. 10.7 g of dry nilotinib dinitrate (V) were obtained with a UPLC chromatographic purity of 98.98%.

### Example 2

Nilotinib base (2 g) was suspended in 10 mL DMSO, and dissolved at 65 °C. The obtained solution was filtered and nitric (V) acid (2 equivalents) in water (w/w 20 %) were added. Into this clear solution 20 mL of methanol was added. Crystallization started at 50 °C and the suspension was cooled to 20 °C. The crystallized material was filtered by vacuum filtration. The wet material was dried under vacuum at 55 °C. 2.16 g of dry nilotinib dinitrate (V) were obtained.

### Example 3

Nilotinib base (2 g) was suspended in DMSO (20 mL). The suspension was heated to 65 °C until a clear solution was obtained and filtered. To the clear solution 2 equivalents of 20% wt% nitric (V) acid in water were added. The solution was cooled to 50°C and methanol (10 mL) was gradually added. Crystallization started at 50 °C and the suspension was cooled to 20 °C within 1 hour. The wet material was dried under vacuum at 55 °C. 2,06 g of dry nilotinib dinitrate (V) were obtained.

### Example 4

Nilotinib base (2 g) was suspended in DMSO (20 mL). The suspension was heated to 65 °C until a clear solution was obtained, and filtered. To the clear solution 2 equivalents of 20% wt% nitric (V) acid in water were added. The clear solution of nilotinb dinitrate (V) salt was added to methanol (10 mL) at 20 °C. Crystallization started at 48 °C and the suspension was cooled to 20 °C in 1 hour. The material was filtered by vacuum filtration. The wet material was dried under vacuum at 55 °C. 2,03 g of dry nilotinib dinitrate (V) were obtained.

### Example 5

Nilotinib base (13 g) was suspended in dimethyl sulphoxide (130 mL) and stirred with overhead stirrer. The suspension was heated to 65 °C until a clear solution was obtained, and filtered. To the clear solution 2 equivalents of 20% wt% nitric (V) acid in water (15,5 g) were added . To the obtained solution methanol (65 mL) was added. Crystallization started at 50 °C and the suspension was cooled to 20 °C in 2 hours. The material was filtered by vacuum filtration and the wet material was dried under vacuum at 55 °C. 12,6g of dry nilotinib dinitrate (V) were obtained.

### Example 6

Nilotinib base (2 g) was suspended in methanol (240 mL) and stirred with a magnetic stirrer. The suspension was heated to 65 °C until a clear solution was obtained, and filtered. To the clear solution 2.0 equivalents of 77% wt% nitric (V) acid in water were added. Crystallization started at 60 °C and the suspension was cooled to 20 °C in 1.5 hour. The material was filtered by vacuum filtration and the wet material was dried under vacuum at 55 °C. 2.3 g of dry nilotinib dinitrate (V) were obtained.

### Example 7

Nilotinib base (2 g) was suspended in mixture of dimethyl sulphoxide and methanol in vol ratio 1:1 (45 mL) and stirred with a magnetic stirrer. The suspension was heated to 65 °C until a clear solution was obtained, and filtered. To the clear solution 2 equivalents of 20% wt% nitric (V) acid in water were added. The solution was cooled to room temperature in 1.5 hour, the crystallization started at 62 °C. The suspension was stirred at room temperature for an additional hour. The material was collected by vacuum filtration and the wet material was dried under vacuum at 55 °C. 2,16 g of dry nilotinib dinitrate (V) were obtained.

### Example 8

Nilotinib base (0.5 g) was suspended in 6.5 mL of THF and stirred with magnetic stirrer. The suspension was heated to 70°C. To the suspension 1.7 ml of water is added to obtain clear solution followed by solution of 2 equivalents of 26.6 % HNO₃ in water. Obtained suspension was cooled to room temperature (crystallisation was started at 60°C) in one hour and stirred at room temperature for one hour. The material was filtered off and vacuum dried at 55°C.

0.5 g of dry nilotinib dinitrate (V) were obtained.

### Example 9

Nilotinib base (0.5 g) was suspended in 8.5 mL of 1-butanol and stirred with magnetic stirrer. The suspension was heated to 100°C. To the suspension 1.7 ml of water is added to obtain clear yellow solution. To obtained solution 2 equivalents of 26.6 % HNO₃ in water is added. Suspension was cooled to room temperature (crystallisation was started at 60°C) in one hour and further stirred at same temperature for one hour. The material was filtered off and vacuum dried at 50°C.

0.54 g of dry nilotinib dinitrate (V) were obtained.

### Example 10

Nilotinib base (0.5 g) was suspended in 5.5 mL of 1,2-propanediol and stirred with magnetic stirrer. The suspension was heated to 100°C to obtain clear solution. To obtained solution 2 equivalents of 26.6 % HNO₃ in water is added. Suspension was cooled to room temperature (crystallisation was started at 70°C) in one hour and further stirred at same temperature for one hour. The material was filtered off and vacuum dried at 50°C.

0.24 g of dry nilotinib dinitrate (V) were obtained.

### Example 11

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of methanol. Suspension was cooled to room temperature (crystallisation was started at 50°C) in one hour and further stirred at same temperature for one hour. The material was filtered off and vacuum dried at 50°C.

1.0 g of dry nilotinib dinitrate (V) was obtained.

### Example 12

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of abs. ethanol. Suspension was cooled to room temperature (crystallisation was started at 50°C) in one hour and further stirred at same temperature for one hour. The material was filtered off and vacuum dried at 50°C.

1.1 g of dry nilotinib dinitrate (V) were obtained.

### Example 13

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of 2-propanol. Suspension was cooled to room temperature (crystallisation was started at 50°C) in one hour and further stirred at same temperature for one hour. The material was filtered off and vacuum dried at 50°C.

1.1 g of dry nilotinib dinitrate (V) were obtained.

### Example 14

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of acetone. Suspension was cooled to room temperature (crystallisation was started at 50°C) in one hour and further stirred at same temperature for one hour. The material was filtered off and vacuum dried at 50°C.

1.1 g of dry nilotinib dinitrate (V) were obtained.

### Example 15

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of ethylmethylketone. Suspension was cooled to room temperature (crystallisation was started at 40°C) in one hour and further stirred at same temperature for one hour. The material was filtered off and vacuum dried at 50°C.

1.1 g of dry nilotinib dinitrate (V) were obtained.

### Example 16

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of methylisobutylketone. Suspension was cooled to room temperature (crystallisation was started at 40°C) in one hour. The material was filtered off and vacuum dried at 50°C.

1.1 g of dry nilotinib dinitrate (V) were obtained.

### Example 17

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of diethylether. Suspension was cooled to room temperature in one hour (crystallisation was started at room temperature) and stirred overnight. The material was filtered off and vacuum dried at 50°C.

1.1 g of dry nilotinib dinitrate (V) were obtained.

### Example 18

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of diisopropylether. Suspension was cooled to room temperature in one hour and stirred overnight. The material was filtered off and vacuum dried at 50°C.

1.1 g of dry nilotinib dinitrate (V) were obtained.

### Example 19

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of THF. Suspension was cooled to room temperature (crystallisation was started at 50°C) in one hour and further stirred at same temperature for one hour. The material was filtered off and vacuum dried at 50°C.

1.1 g of dry nilotinib dinitrate (V) were obtained.

### Example 20

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of ethylacetate. Suspension was cooled to room temperature (crystallisation was started at 70°C) in one hour and further stirred at same temperature for one hour. The material was filtered off and vacuum dried at 50°C.

1.1 g of dry nilotinib dinitrate (V) were obtained.

### Example 21

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of hexane. Suspension was cooled to room temperature (crystallisation was started at room temperature) in one hour and stirred overnight at the same temperature. The material was filtered off and vacuum dried at 50°C.

1.0 g of dry nilotinib dinitrate (V) was obtained.

### Example 22

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of cyclohexane. Suspension was cooled to room temperature (crystallisation was started at room temperature) in one hour and stirred overnight. The material was filtered off and vacuum dried at 50°C.

0.1 g of dry nilotinib dinitrate (V) were obtained.

### Example 23

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of heptane. Suspension was cooled to room temperature (crystallisation was started at room temperature) in one hour and stirred overnight. The material was filtered off and vacuum dried at 50°C.

1.0 g of dry nilotinib dinitrate (V) was obtained.

### Example 24

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of toluene. Suspension was cooled to room temperature (crystallisation was started at 62°C) in one hour and further stirred at same temperature for one hour. The material was filtered off and vacuum dried at 50°C.

1.1g of dry nilotinib dinitrate (V) were obtained.

### Example 25

Nilotinib base (1.0 g) was suspended in 10 mL of DMSO and stirred with magnetic stirrer. The suspension was heated to 77°C to obtain clear solution. To obtained solution 2 equivalents of 26.6 % HNO₃ in water is added, followed by 10 mL of isopropylacetate. Suspension was cooled to room temperature (crystallisation was started at 70°C) in one hour and further stirred at same temperature for one hour. The material was filtered off and vacuum dried at 50°C.

1.1g of dry nilotinib dinitrate (V) were obtained.

### Example 26

Nilotinib base (5.0 g) was suspended in 50 mL of DMSO and stirred with magnetic stirrer. The suspension was heated to 77°C to obtain clear solution. To obtained solution 2 equivalents of 26.6 % HNO₃ in water is added, followed by 50 mL of water. Suspension was cooled to room temperature (crystallisation was started at 60°C) in one hour and further stirred at same temperature for one hour. The material was filtered off and vacuum dried at 50°C.

5.0 g of dry nilotinib dinitrate (V) were obtained.

## Claims

1. Nilotinib dinitrate (V) salt.

2. Nilotinib dinitrate (V) salt according to claim 1, **characterized in that** it is crystalline.

3. A hydrated or anhydrous crystalline form of nilotinib dinitrate (V) according to any of claims 1 to 2.

4. Crystalline nilotinib dinitrate (V) according to any of claims 2 to 3, **characterized by** the following 2-theta degrees: 19.7, 20.7, and 25.8 ±0.2.

5. Crystalline nilotinib dinitrate (V) according to any of claims 2 to 4, further **characterized by** the following 2-theta degrees: 8.9, 18.4, 19.7, 20.7, and 25.8 ±0.2.

6. Crystalline nilotinib dinitrate (V) according to any of claims 2 to 5, further **characterized by** the following 2-theta degrees: 6.5, 8.9, 13.1, 15.6, 18.4, 19.7, 20.7, 23.5, and 25.8 ±0.2.

7. A process for the preparation of nilotinib dinitrate (V) according to claim 1, said process comprising the following steps:
(i) dissolving nilotinib base in inert solvent or mixture of solvents at elevated temperature,
(ii) adding of nitric (V) acid, optionally concentrated, preferably diluted with water, more preferably 20 wt % nitric (V) acid in water,
(iii) optionally contacting the mixture under ii.) with an anti-solvent,
(iv) cooling and optionally stirring the mixture under ii) or iii.) to the crystallization temperature to precipitate the product,
(v) optionally, additionally cooling the suspension to lower temperatures, in particular to optimize the yield and purity of the product,
(vi) isolating and drying the formed solid,
wherein seeding is optionally performed in any of the steps ii) to iv).

8. A process for the preparation of nilotinib dinitrate (V) from nilotinib base according to claim 7, wherein DMSO and/or MeOH are used as solvent or antisolvent.

9. The process for the preparation of nilotinib dinitrate (V) according to claim 7, wherein in step i) the inert solvent or mixture of solvents is selected from the group consisting of dialkylsulfoxides, alcohols, cyclic ethers, and solvent mixtures comprising or consisting of at least two solvents selected from the group consisting of dialkylsulfoxides, alcohols, cyclic ethers, and water.

10. The process for the preparation of nilotinib dinitrate (V) according to claim 9, wherein in step i) the inert solvent or mixture of solvents is selected from the group consisting of dimethylsulfoxide, methanol, butanol, propandiol, tetrahydrofuran, and solvent mixtures comprising or consisting of at least two solvents selected from the group consisting of dimethylsulfoxide, methanol, butanol, propandiol, tetrahydrofuran, and water.

11. The process for the preparation of nilotinib dinitrate (V) according to any one of claims claims 7 to 10, wherein the anti-solvent used in step iii) is an anti-solvent selected from the group of alcohols, ketones, ethers, esters, hydrocarbons, water, and mixtures thereof.

12. The process for the preparation of nilotinib dinitrate (V) according to any one of claims claims 9 to 11, wherein in step i) the inert solvent or mixture of solvents is selected from the group consisting of dialkylsulfoxides, alcohols, cyclic ethers, and solvent mixtures comprising or consisting of at least two solvents selected from the group consisting of dialkylsulfoxides, alcohols, cyclic ethers, and water, and
wherein the anti-solvent used in step iii) is an anti-solvent selected from the group of alcohols, ketones, ethers, esters, hydrocarbons, water, and mixtures thereof.

13. Nilotinib dinitrate (V) salt according to any one of claims 1 to 6 for use as a medicament.

14. Pharmaceutical composition comprising nilotinib dinitrate (V) salt according to any one of claims 1 to 6, and one or more pharmaceutically acceptable excipients.

15. Pharmaceutical composition according to claim 14, which is a solid pharmaceutical composition for oral administration and comprises nilotinib dinitrate (V) salt according to any one of claims 1 to 6, and one or more pharmaceutically acceptable excipients, preferably the solid pharmaceutical composition for oral administration comprises at least one diluent.

## Patentansprüche

1. Nilotinibdinitrat(V)-Salz.

2. Nilotinibdinitrat(V)-Salz gemäß Anspruch 1, **dadurch gekennzeichnet, dass** es kristallin ist.

3. Hydratisierte oder wasserfreie kristalline Form von Nilotinibdinitrat(V) gemäß einem der Ansprüche 1 bis 2.

4. Kristallines Nilotinibdinitrat(V) gemäß einem der Ansprüche 2 bis 3, **gekennzeichnet durch** die folgenden 2-theta Grade: 19,7, 20,7 und 25,8 ± 0,2.

5. Kristallines Nilotinibdinitrat(V) gemäß einem der Ansprüche 2 bis 4, ferner **gekennzeichnet durch** die folgenden 2-theta Grade: 8,9, 18,4, 19,7, 20,7 und 25,8 ± 0,2.

6. Kristallines Nilotinibdinitrat(V) gemäß einem der Ansprüche 2 bis 5, ferner **gekennzeichnet durch** die folgenden 2-theta Grade: 6,5, 8,9, 13,1, 15,6, 18,4, 19,7, 20,7, 23,5 und 25,8 ± 0,2.

7. Verfahren zur Herstellung von Nilotinibdinitrat(V) gemäß Anspruch 1, wobei das Verfahren die folgenden Schritte umfasst:
(i) Lösen von Nilotinib-Base in inertem Lösungsmittel oder Gemisch von Lösungsmitteln bei erhöhter Temperatur,
(ii) Zugeben von Salpeter(V)-Säure, gegebenenfalls konzentriert, bevorzugt verdünnt mit Wasser, stärker bevorzugt 20 Gew.-%iger Salpeter(V)-Säure in Wasser,
(iii) gegebenenfalls Inkontaktbringen des Gemisches unter ii) mit einem Anti-Lösungsmittel,
(iv) Kühlen und gegebenenfalls Rühren des Gemisches unter ii) oder iii) auf die Kristallisationstemperatur, um das Produkt auszufällen,
(v) gegebenenfalls zusätzliches Kühlen der Suspension auf niedrigere Temperaturen, insbesondere um die Ausbeute und Reinheit des Produkts zu optimieren,
(vi) Isolieren und Trocknen des gebildeten Feststoffes,
wobei Animpfen gegebenenfalls in jedwedem der Schritte ii) bis iv) durchgeführt wird.

8. Verfahren zur Herstellung von Nilotinibdinitrat(V) aus Nilotinib-Base gemäß Anspruch 7, wobei DMSO und/oder MeOH als Lösungsmittel oder Antilösungsmittel verwendet werden.

9. Verfahren zur Herstellung von Nilotinibdinitrat(V) gemäß Anspruch 7, wobei in Schritt i) das inerte Lösungsmittel oder Gemisch von Lösungsmitteln aus der Gruppe ausgewählt ist, welche aus Dialkylsulfoxiden, Alkoholen, cyclischen Ethern und Lösungsmittelgemischen, umfassend oder bestehend aus mindestens zwei Lösungsmitteln, die aus der Gruppe ausgewählt sind, welche aus Dialkylsulfoxiden, Alkoholen, cyclischen Ethern und Wasser besteht, besteht.

10. Verfahren zur Herstellung von Nilotinibdinitrat(V) gemäß Anspruch 9, wobei in Schritt i) das inerte Lösungsmittel oder Gemisch von Lösungsmitteln aus der Gruppe ausgewählt ist, welche aus Dimethylsulfoxid, Methanol, Butanol, Propandiol, Tetrahydrofuran und Lösungsmittelgemischen, umfassend oder bestehend aus mindestens zwei Lösungsmitteln, die aus der Gruppe ausgewählt sind, welche aus Dimethylsulfoxid, Methanol, Butanol, Propandiol, Tetrahydrofuran und Wasser besteht, besteht.

11. Verfahren zur Herstellung von Nilotinibdinitrat(V) gemäß einem der Ansprüche 7 bis 10, wobei das in Schritt iii) verwendete Anti-Lösungsmittel ein Anti-Lösungsmittel, ausgewählt aus der Gruppe von Alkoholen, Ketonen, Ethern, Estern, Kohlenwasserstoffen, Wasser und Gemischen davon, ist.

12. Verfahren zur Herstellung von Nilotinibdinitrat(V) gemäß einem der Ansprüche 9 bis 11, wobei in Schritt i) das inerte Lösungsmittel oder Gemisch von Lösungsmitteln aus der Gruppe ausgewählt ist, welche aus Dialkylsulfoxiden, Alkoholen, cyclischen Ethern und Lösungsmittelgemischen, umfassend oder bestehend aus mindestens zwei Lösungsmitteln, die aus der Gruppe ausgewählt sind, welche aus Dialkylsulfoxiden, Alkoholen, cyclischen Ethern und Wasser besteht, besteht, und
wobei das in Schritt iii) verwendete Anti-Lösungsmittel ein Anti-Lösungsmittel, ausgewählt aus der Gruppe von Alkoholen, Ketonen, Ethern, Estern, Kohlenwasserstoffen, Wasser und Gemischen davon, ist.

13. Nilotinibdinitrat(V)-Salz gemäß einem der Ansprüche 1 bis 6 zur Verwendung als ein Medikament.

14. Pharmazeutische Zusammensetzung, umfassend Nilotinibdinitrat(V)-Salz gemäß einem der Ansprüche 1 bis 6 und einen oder mehr pharmazeutisch verträgliche Exzipienten.

15. Pharmazeutische Zusammensetzung gemäß Anspruch 14, welche eine feste pharmazeutische Zusammensetzung für orale Verabreichung ist und Nilotinibdinitrat(V)-Salz gemäß einem der Ansprüche 1 bis 6 und einen oder mehr pharmazeutisch verträgliche Exzipienten umfasst, wobei die feste pharmazeutische Zusammensetzung für orale Verabreichung bevorzugt mindestens ein Verdünnungsmittel umfasst.

## Revendications

1. Sel de dinitrate de nilotinib (V).

2. Sel de dinitrate de nilotinib (V) selon la revendication 1, **caractérisé en ce qu'**il est cristallin.

3. Forme cristalline hydratée ou anhydre de dinitrate de nilotinib (V) selon l'une des revendications 1 et 2.

4. Dinitrate de nilotinib (V) cristallin selon l'une des revendications 2 et 3, **caractérisé par** les degrés 2-thêta suivants : 19,7, 20,7 et 25,8 ± 0,2.

5. Dinitrate de nilotinib (V) cristallin selon l'une des revendications 2 à 4, **caractérisé en outre par** les degrés 2-thêta suivants : 8,9, 18,4, 19,7, 20,7 et 25,8 ± 0,2.

6. Dinitrate de nilotinib (V) cristallin selon l'une des revendications 2 à 5, **caractérisé en outre par** les degrés 2-thêta suivants : 6,5, 8,9, 13,1, 15,6, 18,4, 19,7, 20,7, 23,5 et 25,8 ± 0,2.

7. Procédé de préparation de dinitrate de nilotinib (V) selon la revendication 1, ledit procédé comprenant les étapes suivantes qui consistent :
(i) à dissoudre une base de nilotinib dans un solvant ou mélange de solvants inerte à température élevée,
(ii) à ajouter de l'acide nitrique (V), éventuellement concentré, de préférence dilué avec de l'eau, plus préférablement 20% en poids d'acide nitrique (V) dans l'eau,
(iii) à mettre éventuellement en contact le mélange de ii) avec un anti-solvant,
(iv) à refroidir et éventuellement agiter le mélange de ii) ou iii) à la température de cristallisation pour précipiter le produit,
(v) éventuellement, à refroidir davantage la suspension à des températures plus basses, notamment pour optimiser le rendement et la pureté du produit,
(vi) à isoler et sécher le solide formé,
Procédé dans lequel la germination est éventuellement effectuée dans l'une des étapes ii) à iv).

8. Procédé de préparation de dinitrate de nilotinib (V) à partir d'une base de nilotinib selon la revendication 7, dans lequel du DMSO et/ou du MeOH sont utilisés comme solvant ou anti-solvant.

9. Procédé de préparation de dinitrate de nilotinib (V) selon la revendication 7, dans lequel à l'étape i) le solvant ou mélange de solvants inerte est choisi dans le groupe constitué de dialkylsulfoxydes, d'alcools, d'éthers cycliques et de mélanges de solvants comprenant ou constitués d'au moins deux solvants choisis dans le groupe constitué de dialkylsulfoxydes, d'alcools, d'éthers cycliques et de l'eau.

10. Procédé de préparation de dinitrate de nilotinib (V) selon la revendication 9, dans lequel à l'étape i) le solvant ou mélange de solvants inerte est choisi dans le groupe constitué de diméthylsulfoxyde, de méthanol, de butanol, de propandiol, de tétrahydrofurane et de mélanges de solvants comprenant ou constitués d'au moins deux solvants choisis dans le groupe constitué de diméthylsulfoxyde, de méthanol, de butanol, de propandiol, de tétrahydrofurane et de l'eau.

11. Procédé de préparation de dinitrate de nilotinib (V) selon l'une quelconque des revendications 7 à 10, dans lequel l'anti-solvant utilisé à l'étape iii) est un anti-solvant choisi dans le groupe constitué d'alcools, de cétones, d'éthers, d'esters, d'hydrocarbures, de l'eau et de leurs mélanges.

12. Procédé de préparation de dinitrate de nilotinib (V) selon l'une quelconque des revendications 9 à 11, dans lequel à l'étape i) le solvant ou mélange de solvants inerte est choisi dans le groupe constitué de dialkylsulfoxydes, d'alcools, d'éthers cycliques, et de mélanges de solvants comprenant ou constitués d'au moins deux solvants choisis dans le groupe constitué de dialkylsulfoxydes, d'alcools, d'éthers cycliques et de l'eau, et
dans lequel l'anti-solvant utilisé à l'étape iii) est un anti-solvant choisi dans le groupe constitué d'alcools, de cétones, d'éthers, d'esters, d'hydrocarbures, de l'eau et de leurs mélanges.

13. Sel de dinitrate de nilotinib (V) selon l'une quelconque des revendications 1 à 6 pour une utilisation comme médicament.

14. Composition pharmaceutique comprenant du sel de dinitrate de nilotinib (V) selon l'une quelconque des revendications 1 à 6, et un ou plusieurs excipient(s) pharmaceutiquement acceptable(s).

15. Composition pharmaceutique selon la revendication 14, qui est une composition pharmaceutique solide pour administration orale et comprend du sel de dinitrate de nilotinib (V) selon l'une quelconque des revendications 1 à 6, et un ou plusieurs excipient(s) pharmaceutiquement acceptable(s), de préférence la composition pharmaceutique solide pour administration orale comprend au moins un diluant.
